# EUROPEAN PATENT SPECIFICATION

(11) **EP 3 017 040 B1**
(45) Date of publication and mention of the grant of the patent: **06.06.2018**
(21) Application number: 14733650.7
(22) Date of filing: 30.06.2014
(51) Int. Cl.: C12N 7/02, A61K 39/145, A61K 39/12, A61K 39/295, C12N 7/00, A61K 39/00

(54) **METHOD FOR PREPARING VIROSOMES**
VERFAHREN ZUR HERSTELLUNG VON MULTIVALENTEN VIROSOMEN
PROCÉDÉ DE PRÉPARATION DE VIROSOMES MULTIVALENTS

(30) Priority: 02.07.2013 EP 13174696
(43) Date of publication of application: 11.05.2016
(73) Proprietor: Janssen Vaccines & Prevention B.V., 2333 CN Leiden (NL)
(72) Inventor: DONG, Wenyu, 2333 CN Leiden (NL); RIJKEN, Pieter, 2333 CN Leiden (NL); UGWOKE, Mike, 2333 CN Leiden (NL)
(74) Representative: Manten, Annemieke
(86) International application number: PCT/EP2014/063814
(87) International publication number: WO 2015/000832

(56) References cited:
- WO-A2-2007/130330
- US-B2- 7 901 920
- MENGIARDI B ET AL: "Virosomes as carriers for combined vaccines", VACCINE, ELSEVIER LTD, GB, vol. 13, no. 14, 1 October 1995 (1995-10-01), pages 1306-1315, XP004057432, ISSN: 0264-410X, DOI: 10.1016/0264-410X(95)00028-Y
- MISCHLER R ET AL: "Inflexal<(>R)V a trivalent virosome subunit influenza vaccine: production", VACCINE, ELSEVIER LTD, GB, vol. 20, 20 December 2002 (2002-12-20), pages B17-B23, XP004397481, ISSN: 0264-410X, DOI: 10.1016/S0264-410X(02)00512-1
- GLUECK R ET AL: "IMMUNOGENICITY OF NEW VIROSOME INFLUENZA VACCINE IN ELDERLY PEOPLE", LANCET, LITTLE, BROWM AND CO., BOSTON, US, vol. 344, no. 8916, 16 July 1994 (1994-07-16), pages 160-163, XP008023142, ISSN: 0099-5355, DOI: 10.1016/S0140-6736(94)92758-8
- WILSCHUT ET AL: "Influenza vaccines: The virosome concept", IMMUNOLOGY LETTERS, ELSEVIER BV, NL, vol. 122, no. 2, 21 February 2009 (2009-02-21), pages 118-121, XP026029710, ISSN: 0165-2478, DOI: 10.1016/J.IMLET.2008.11.006 [retrieved on 2009-02-21]

## Description

The invention relates to the field of medicine and in particular to the field of infectious diseases. More in particular, the invention relates to methods for preparing virosome preparations and to the virosome preparations obtainable thereby. The invention furthermore relates to novel virosomes, to immunogenic compositions that include the virosomes, and to uses thereof, e.g. in vaccines.

### Background

Virosomes are reconstituted phospholipid (PL) membranes containing proteins from a virus, like hemagglutinin (HA) and neuraminidase (NA) from an influenza virus. These virosomes are derived from influenza viruses, or from other enveloped viruses, such as, but not limited to the following families: flaviviridae (e.g. Dengue virus, Hepatitis C virus HEV, Japanese encephalitis virus, Yellow fever virus, West Nile virus), Poxyiridae (e.g.. Cowpox virus, Monkeypox virus, vaccinia virus, Variola virus), Retroviridae (e.g. Immuno deficiency viruses HIV/SIV, paramyxoviridae (e.g.. Measles virus, Mumps virus, Parainfluenza viruses, metapneumovirus, Respiratory Syncytial virus RSV), and Orthomyxoviridae (e.g. Influenza viruses). Virosomes generally are prepared from such enveloped viruses by solubilisation of the viral envelope and removal of the nucleocapsid containing the viral genome, followed by reconstitution of an "empty" viral envelope comprising a lipid membrane containing viral glycoproteins derived from the enveloped virus, such as hemagglutinin (HA) in the case of influenza virosomes. Virosomes can be produced by either the addition of exogenous lipids (e.g. as described in US2009/0263470 and US2009/0087453) or without the addition of exogenous lipids (as described in US7901920). Additional antigens may be adhered to the virosomes, or cross-linked to lipid molecules in order to anchor them in the virosomal membrane.

Since virosomes do not contain the viral genomic material (e.g. viral genomic RNA or DNA), they are nonreplicative in nature, which make them safe for administration to animals and humans in the form of an immunogenic composition (e.g. as a vaccine), or as an adjuvant. Virosomes thus have been especially useful in the field of vaccination, where it is desired to stimulate an immune response to an antigen associated with a particular disease or disorder. In such cases, an antigen is typically encapsulated in or bound to the virosome, which then delivers this antigen to the host immune system to be vaccinated. By virtue of the particular antigen delivered, the resulting prophylactic and/or therapeutic effect is necessarily specific for the disease or disorder with which the antigen is associated. Thus, influenza virosomes that essentially are reconstituted virus envelopes containing the hemagglutinin antigen incorporated in a phospholipid membrane envelope, but without having the genetic background genome of the influenza virus itself, have been approved for seasonal influenza vaccination (marketed under the trademark Inflexal® V).

Infections with influenza viruses are associated with a broad spectrum of illnesses and complications that result in substantial worldwide morbidity and mortality, especially in the elderly and patients with chronic illnesses. Vaccination against influenza is most effective in preventing the possible fatal complications associated with this infection, and much effort has been put in the development of influenza vaccines, including those based on virosome technology. The yearly trivalent influenza vaccine typically contains antigens from two influenza A virus strains and one influenza B virus strain. Based on the epidemiological surveys by over hundred National Influenza Centers worldwide, the World Health Organization (WHO) yearly recommends the composition of the influenza vaccine, usually in February for the Northern hemisphere, and in September for the Southern hemisphere. In this context, this practice limits the time window for production and standardization of the influenza vaccine is limited.

The current process for the manufacture of the trivalent virosomal influenza vaccine involves multiple processes which make the process less efficient and increase the cost of goods. In addition, due to the fact that multiple process steps are involved, there is a relatively high risk of contamination occurring during the manufacturing process. Furthermore, due to the increasing world population, growing and emerging economies, intensified international traveling, the growing spread of yearly influenza epidemics, and the threat of worldwide influenza pandemics, the demand for influenza vaccines is still growing. There is thus an ongoing need for simplified and/or improved methods for preparing virosome preparations..

### Summary of the invention

The present invention provides a novel method for preparing a virosome preparation comprising viral envelope protein of at least two, preferably at least three, more preferably at least four different enveloped viruses. The method of the invention comprises the steps of:
(a) providing at least two, preferably at least three, more preferably at least four enveloped viruses and optionally inactivating said at least two, preferably at least three, more preferably at least four enveloped viruses;
(b) solubilizing the at least two, preferably at least three, more preferably at least four viral envelopes by adding a solubilizing agent; and
(c) reconstituting virosomal membranes comprising viral envelope protein of said at least two, preferably at least three, more preferably at least four different viruses by removing the solubilizing agent,
wherein the viral envelopes of said two, preferably at least three, more preferably at least four different virus are blended prior to reconstitution of the virosomal membranes.

The present disclosure further provides virosomes having a virosomal membrane comprising viral envelope protein of at least two, preferably at least three, more preferably at least four different viruses.

The enveloped virus preferably is an influenza virus, and the at least two, three, or four different viruses preferably are two, three, four or more different influenza A and/or B virus strains. However, the method of the invention also applies to all other envelope viruses that can be used to prepare virosomes.

The disclosure also provides compositions, preferably immunogenic compositions, comprising said virosome preparations or virosomes.

The disclosure also relates to (immunogenic) compositions comprising the virosomes or virosomal preparations, and the use thereof e.g. for eliciting and/or increasing an immune response. In a preferred embodiment, the composition is a vaccine. The compositions are preferably suitable for human administration. The compositions may be used in methods of preventing and/or treating any viral disease, in particular but not limited to those caused by influenza virus. The invention further relates to the use of the virosomes as a vaccine and/or as an adjuvant .The invention in particular relates to the use of the virosomes as a vaccine for the prevention and/or treatment of influenza.

### Figures

FIG. 1 is a process flow chart showing the procedure of preparing monovalent virosome particles of the prior art.
FIG. 2 shows blending of three monovalent virosomal preparations into a trivalent virosomal preparation according to the prior art.
FIG. 3 is a process flow chart for the preparation of a virosome preparation according to the invention.
FIG. 4 shows the virosome particles prepared using the method of the prior art (A) and using the method of the present invention (B).

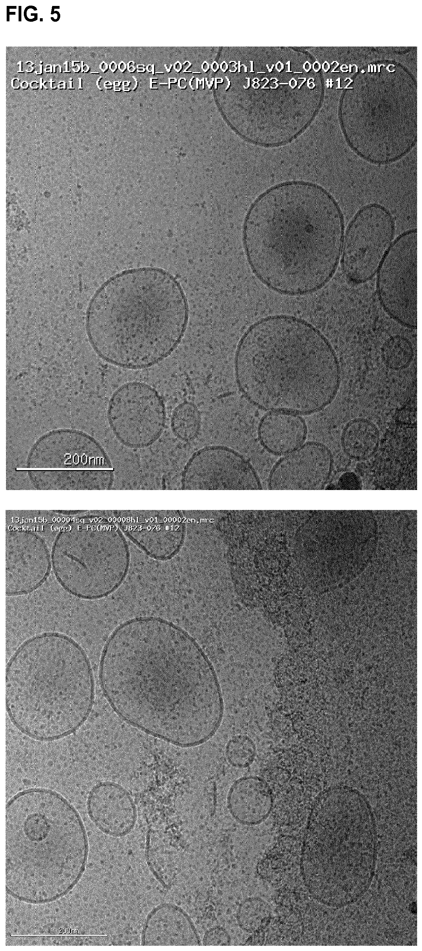

FIG 5 shows a CryoTEM micrograph of cocktail virosomes made according to the current invention, showing optically-dense regions in the virosome membrane indicating regions of protein (HA and NA) insertion in the membranes.

### Detailed description of the invention

The present invention provides a novel and improved method for preparing virosome preparations comprising viral envelope protein of at least two, preferably at least three, more preferably at least four different enveloped viruses, comprising the steps of:
(a) providing at least two, preferably at least three, more preferably at least four enveloped viruses and optionally inactivating said at least two, preferably at least three, more preferably at least four enveloped viruses;
(b) solubilizing the at least two, preferably at least three, more preferably at least four viral envelopes by adding a solubilizing agent; and
(c) reconstituting virosomal membranes comprising viral envelope protein of said at least two, preferably at least three, more preferably at least four different viruses by removing the solubilizing agent,
wherein the viral envelopes of said two, preferably at least three, more preferably at least four different virus are blended prior to reconstitution of the virosomal membranes.

According to the invention, a simplified method for preparing multivalent virosomal preparations, in particular virosome preparations comprising virosomes having a virosomal membrane comprising viral envelope protein of two or more different enveloped viruses, in particular viral envelope protein of at least three, preferably at least four different enveloped viruses is provided. The term virosomal membrane" as used herein refers to a membrane structure that is reconstituted in vitro and that is composed of a lipid bilayer with integrated viral envelope proteins. The term "envelope protein" refers to protein that is encoded by an enveloped virus and that is normally associated with or embedded in the viral envelope.

In certain embodiments, the method further comprises a step of adding exogenous lipids. By "exogenous lipids" lipids are meant that are not endogenous to enveloped virus but that are added to the virus components during preparation of the virosomes. According to the invention a broad range of lipids can be incorporated in the virosomal membrane. The preferred virosomal preparations are based on phospholipids, such as but not limited to egg-derived phospholipids in order to minimize the complexity of the formulation.

In certain embodiments, the method further comprises the step of adding further exogenous components. The term "exogenous component" according to the invention means any additional component that is not endogenous to the virus but is added to the virus components during preparation of the virosomes. Exogenous components that may be added according to the invention include lipoproteins, lipopolysaccharides, adjuvants and/or targeting ligands.

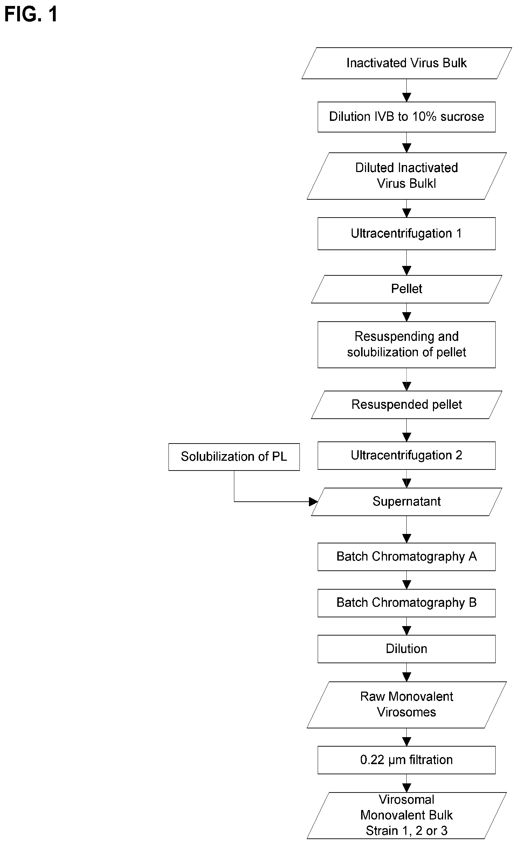

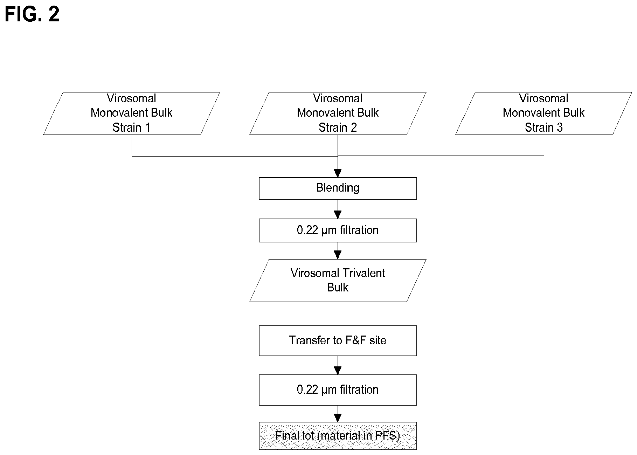

Multivalent virosomal compositions are known, i.e. vaccine compositions containing different virosomes each with the viral envelope proteins of a single virus strain , such as the virosomal influenza vaccine Inflexal V®, which typically contains hemagglutinin (HA) from two influenza A virus strains, generally an influenza A H1N1 and an H3N2 strain and an influenza B strain. Such multivalent virosomal compositions typically are produced by preparing monovalent virosome preparations from the different influenza virus strains and subsequently blending the monovalent virosome preparations into a multivalent virosome preparation. Figure 1 schematically shows the current process steps for producing an influenza virosomal vaccine. In short, the virosomal vaccine is produced by first making individual virosome monovalent bulk (VMVB) from each virus strain by multiple steps. Subsequently, the VMVBs from different virus strains are blended into trivalent virosome preparations (3 strains), or optionally quadrivalent preparations (4 strains), prior to filling in final containers as the final drug product (Figure 2).

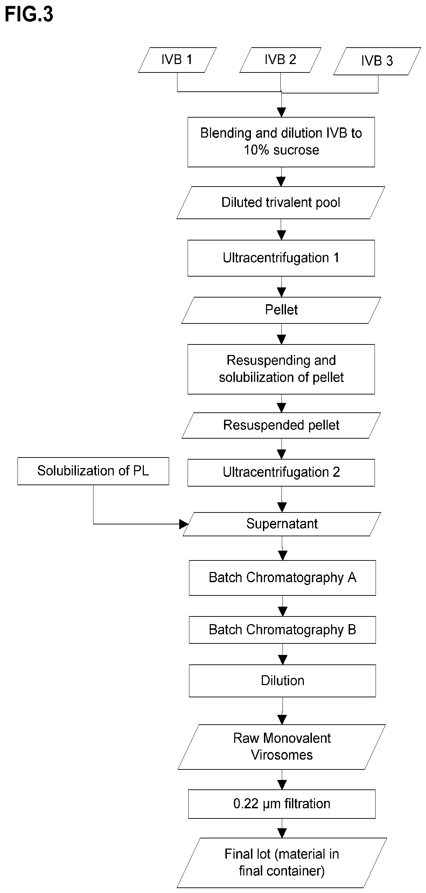

According to the invention, it has surprisingly been shown that this method could be simplified substantially by combining several process flows into one. Thus, as shown in Figure 3, whole viruses, such as influenza viruses (either activated or inactivated), or the supernatants after solubilization of the viral membranes with solubilizing solutions, of 2, 3, 4, or more different viruses or virus strains are blended. Subsequently, the blended virus bulk or the supernatant can be be processed in one single flow to achieve the final multivalent virosomal preparation, such as e.g. a trivalent or quadrivalent virosome product. Prior to the invention, it was generally thought by people skilled in the art, that the method as described herein could lead to lop-sided incorporation of HA into the bilayer. In addition, the different stability of different influenza strains could lead to differences in stability during processing and thus to product of less quality. Surprisingly, however, it was demonstrated that a random and equitable distribution of HA from different virus strains per virosome particle was obtained, and no negative effect on product quality was observed.

The influenza viruses may be incativated. According to the invention, inactivation of the viruses may be accomplished using methods well-known in the art, e.g. by use of formaldehyde or beta-propiolactone (BPL).

Solubilization of the viral envelopes is achieved by adding a solubilizing agent. Suitable solublizing agents are known in the art. Preferably, the solubilizing agent is a non-ionic detergent. Examples for non-ionic detergents according to the invention comprise detergents e.g. such as octaethylene glycol mono(N-dodecyl)ether (OEG), Triton X-100, Triton X-114, NP 40 and, Polysorbate 20 or 80. The detergents may be preferably used in a concentration range of 10-1000 mM. In certain embodiments, the non-ionic detergent is octaethylene glycol monododecyl ether (OEG).

After solubilization of the viral envelopes and optionally addition of exogenous lipids, and/or further exogenous components such as, but not limited to adjuvants, targeting ligands, the virosomal membranes are reconstituted by removing the solubilizing agent. Removing the solubilizing agent according to the invention may be accomplished by processes such as dialysis, diafiltration, or chromatography. The latter, chromatography, which is based on eliminating detergent by adsorbance to a matrix (e.g. beads, resin) is preferred.

In certain embodiments, the viral envelopes of said at least said two, preferably at least three, more preferably at least four enveloped viruses are blended prior inactivating the at least two, three, four enveloped viruses. In such embodiments, the different whole viruses thus are blended and subjected to a simultaneous virus inactivation step.

In a further embodiment, the viral envelopes of said at least said two preferably at least three, more preferably at least four enveloped viruses are blended after inactivation of the viruses and prior to solubilizing the viral envelopes. The individual viruses thus are inactivated individually prior to blending them.

In yet another embodiment, the viral envelopes of said at least said two preferably at least three, more preferably at least four enveloped viruses are blended after (separately) solubilizing the viral envelopes. Thus, preparations comprising solubilized viral envelopes of said at least two, preferably at least three, more preferably at least four different viruses, are blended, prior to reconstitution of the viral envelopes.

Viruses, such as e.g. influenza viruses, generally contain several virus strains which usually result from mutations and which may show different pathogenic profiles. These different strains can be combined in order to provide multivalent, e.g. trivalent, vaccines. In certain embodiments, the at least two, preferably at least three, more preferably at least four different enveloped viruses thus comprise at least two, preferably at least three, more preferably at least four different virus strains of a specific type or species of an enveloped virus. In a particular embodiment, the enveloped virus is an influenza virus and the envelope protein is hemagglutinin (HA) and/or neuraminidase (NA). The at least two, three, or four virus strains thus may comprise several influenza A virus strains, such as e.g. H1N1 and/or H3N2 influenza virus strains, and/or several influenza B virus strains. Other enveloped viruses may however also be used according to the present invention.

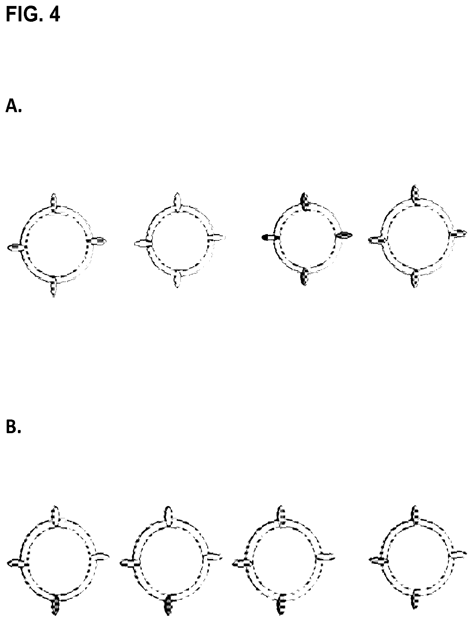

According to the present invention, it has been shown that the structure of the virosome particles obtained by the method described herein is different from the virosome particles obtained using methods of the prior art. Thus, envelope protein (e.g. HA) from different viruses or virus strains is spiked in the same virosome particle (Figure 4B) yielding a homogenous distribution of envelope protein (e.g. HA) from different viruses or virus strains among all virosome particles. This is contrary to the current situation involving blending of e.g. 3 or 4 different virosome preparations, wherein the final virosomal preparation is a heterogeneous mixture of 3 or 4 monovalent virosome types (Figure 4A). Since in vitro stability of the final multivalent virosome preparations generally is virus strain dependent, this needs to be improved for each virus strain in order to have a similar stability profile. With the method according to the invention, a stable virosome preparation is obtained. Virosome preparations obtainable by the method of the present invention thus also form part of the present invention.

In certain embodiments, the method may further comprise a step of adding one or more additional antigens. The antigen(s) may be incorporated into the virosome (e.g. contained in its lumen), absorbed to/bound to the surface of the virosome, integrated into the lipid membrane of the virosome, and the like.

In certain embodiments, the method further comprises the step of purifying the reconstitured virosomes. Purification of the virosomes may be accomplished using standard techniques known in the art, such as, but not limted to batch chromatography with e.g. Biobeads®), affinity chromatography, gel filtration, dialysis, cross-flow filtration etc.

In certain embodiments, the method further comprises the step of size reduction of the reconstituted virosomes. Size reduction may be accomplished by techniques, such as, but not limited to (high pressure) homogenization, and extrusion.

The disclosure further relates to virosome particles having a virosomal membrane comprising viral envelope protein of at least two, preferably at least three, more preferably at least four different enveloped viruses. In a preferred embodiment, the virosomes comprise envelope protein of at least two preferably at least three, more preferably at least four different virus strains of an enveloped virus. In a particular embodiment, the enveloped virus is an influenza virus and the viral envelope protein is HA and/or NA.

In certain embodiments, the virosomes comprise envelope protein of at least three different virus strains, in particular at least three different influenza virus strains, such as e.g. two influenza A virus strains, such as e.g. H1N1 and/or H3N2 influenza virus strains, and one influenza B virus strain. In another embodiment, the virosomes comprise envelope protein of at least four different virus strains, in particular at least four different influenza virus strains, such as e.g. two influenza A virus strains, such as e.g. H1N1 and/or H3N2 influenza virus strains, and two influenza B virus strains.

In further embodiments, the virosomes further comprise one or more antigens. Thus, the virosomes may comprise one or more additional antigens incorporated in the virosomal membrane and/or linked thereto. The antigen may be incorporated into the virosome (e.g. contained in its lumen), absorbed to/bound to the surface of the virosome, integrated into the lipid membrane of the virosome, and the like. A virosome loaded with antigen may be used as an antigen delivery vehicle.

The disclosure furthermore relates to compositions comprising the above described virosome preparations or virosomes. The disclosure further provides immunogenic compositions comprising a therapeutically effective amount of the virosome preparations or virosomes. The (immunogenic) compositions may further comprise a pharmaceutically acceptable excipient. By "pharmaceutically acceptable excipient" is meant any inert substance that is combined with an active molecule for preparing an agreeable or convenient dosage form. The "pharmaceutically acceptable excipient" preferably is an excipient that is non-toxic to recipients at the used dosages and concentrations, and is compatible with other ingredients of the formulation. Pharmaceutically acceptable excipients are widely applied and known in the art (see Remington's Pharmaceutical Sciences, 18th edition, A. R. Gennaro, Ed., Mack Publishing Company [1990]; Pharmaceutical Formulation Development of Peptides and Proteins, S. Frokjaer and L. Hovgaard, Eds., Taylor & Francis [2000]; and Handbook of Pharmaceutical Excipients, 3rd edition, A. Kibbe, Ed., Pharmaceutical Press [2000]). The exact formulation should suit the mode of administration.

In certain embodiments, the immunogenic compositions may comprise, or are administered in combination with, an adjuvant. The adjuvant may be administered before, concomitantly with, or after administration of said composition. Examples of suitable adjuvants include saponin formulations, such as, but not limited to QS21, immunostimulating complexes (ISCOMs) (see e.g. US 5,057,540; WO 90/03184, WO 96/11711, WO 2004/004762, WO 2005/002620); bacterial or microbial derivatives, examples of which are monophosphoryl lipid A (MPL), 3-O-deacylated MPL (3dMPL), CpG-motif containing oligonucleotides, ADP-ribosylating bacterial toxins or mutants thereof, such as E. coli heat labile enterotoxin LT, cholera toxin CT, pertussis toxin PT, or tetanus toxoid TT, Matrix M and cationic lipids. However, since virosomes have inherent adjuvanting activity, in certain embodiments, the immunogenic compositions of the present invention do not comprise additional adjuvants.

The disclosure also relates to methods for inducing and/or increasing an immune response in a subject, the method comprising administering to a subject the above-described virosomal preparations or immunogenic compositions. According to the invention, the induced immune response may be directed against the viral envelope protein and/or to the additional (viral) antigen(s). A subject according to the invention preferably is a mammal that is capable of being infected with an infectious disease-causing virus, in particular an influenza virus. Preferably, the subject is a human subject.

In certain embodiments, the disclosure provides methods for inducing an immune response to an influenza virus envelope protein, such as HA and/or NA. In some embodiments, the immune response induced is effective to prevent and/or treat an influenza virus infection.

The term "therapeutically effective amount" as described herein refers to an amount of virosomes that is effective for preventing, ameliorating and/or treating a condition resulting from infection with a virus, in particular, but not limited to an influenza virus. Ameloriation as used in herein may refer to the reduction of visible or perceptible disease symptoms, viremia, or any other measurable manifestation of the viral infection, in particular the influenza infection. Prevention encompasses inhibiting or reducing the spread of virus or inhibiting or reducing the onset, development or progression of one or more of the symptoms associated with infection with the virus, in particular influenza virus.

The disclosure also relates to the above-described compositions and/or immunogenic compositions for inducing and/or increasing an immune response in a subject, in particular for use as a vaccine and/or as an adjuvant. The virosomal preparations, virosome particles and/or immunogenic compositions thus may be used to elicit neutralizing antibodies against the viral envelope protein, e.g. to HA and/or NA of influenza virus and/or to the additional antigen(s). The invention in particular relates to the virosomal preparations, virosomes and/or immunogenic compositions as described above for use as a vaccine in the prevention and/or treatment of a disease or condition caused by an influenza A and/or B virus.

Administration of the virosomal preparations and/or (immunogenic) compositions can be performed using standard routes of administration. Non-limiting examples include parenteral administration, such as intravenous, intradermal, transdermal, intramuscular, subcutaneous, or mucosal administration, e.g. intranasal, oral, and the like. In certain embodiments, the virosomal preparations and/or immunogenic compositions are administered more than one time, i.e. in a so-called homologous prime-boost regimen. In certain embodiments where the virosomal preparations and/or immunogenic compositions are administered more than once, the administration of the second dose can be performed after a time interval of, for example, one week or more after the administration of the first dose, two weeks or more after the administration of the first dose, three weeks or more after the administration of the first dose, one month or more after the administration of the first dose, six weeks or more after the administration of the first dose, two months or more after the administration of the first dose, 3 months or more after the administration of the first dose, 4 months or more after the administration of the first dose, etc, up to several years after the administration of the first dose of the virosomal preparations and/or immunogenic compositions. It is also possible to administer the virosomal preparations and/or immunogenic compositions more than twice, e.g. three times, four times, etc, so that the first priming administration is followed by more than one boosting administration. In other embodiments, the virosomal preparations and/or immunogenic compositions according to the invention are administered only once. The virosomal preparations and/or immunogenic compositions may also be administered, either as prime, or as boost, in a heterologous prime-boost regimen.

In certain embodiments, prevention and/or treatment may be targeted at patient groups that are susceptible to infection with the virus, in particular to influenza virus infection. Such patient groups include, but are not limited to e.g., the elderly (e.g. ≥ 50 years old, ≥ 60 years old, and preferably ≥ 65 years old), the young (e.g. ≤ 5 years old, ≤ 1 year old), hospitalized patients and patients who have been treated with an antiviral compound but have shown an inadequate antiviral response.

In other embodiments, the virosomal preparations immunogenic compositions may be administered to a subject in combination with one or more other active agents, such as existing vaccines, monoclonal antibodies and/or antiviral agents, and/or antibacterial, and/or immunomodulatory agents. In another embodiment, other active agents can be incorporated into the virosome core or bilayer.

The present invention is further illustrated in the following Examples.

### Example 1. Preparation of trivalent influenza virosomal preparation according to the invention

Egg-based and PER.C6 cell-based trivalent virosomal preparation were prepared according to the invention, after blending inactivated viruses.

### Solubilization of the inactivated virus bulk and phospholipids

The inactivated virus bulks (IVB, A. California, A. Victoria and B. Brisbane) were pooled, diluted with phosphate-buffered saline (PBS, pH 7.4), mixed with a magnetic stirrer and divided into ultracentrifugation bottles, followed by ultracentrifugation at >15000rpm for 1 hour. In this example the experimental starting ratio of the IVBs was 0,2: 0,4: 0,3 for A. California, A. Victoria and B. Brisbane, respectively . The supernatants of all the UC bottles/tubes were discarded and OEG solution (55% of required volume) serially added to resuspend the pellet in different bottles. The ultracentrifugation bottles/tubes were rinsed serially with additional OEG solution (35% of required volume), and pooled with the first suspension and the resuspended pellet stirred overnight at 4°C. The resuspended pellet was divided in the ultracentrifugation bottles/tubes and each bottles/tubes and centrifuged at 28000rpm or higher.Gently the supernatant was withdrawn from the bottle/tube and poured into a clean glass bottle without disturbing the pellet in the bottom of the tube. The phospholipids (PL) were weighed directly into a clean glass vial and the OEG solution (10% of required volume) added and mixed with magnetic stirrer overnight at 4°C for solubilization. The PL suspension and supernatant containing viral envelope material were combined and mixed for 30 - 60 minutes until the mixture was free of any visible particles.

### Preparation of Biobeads®

The Biobeads® were weighed into a glass flask, methanol added and stirred for 10 minutes followed by filtration to remove the methanol. This was repeated with demineralized water. Residual moisture content of 1 gram of Biobeads® was determined using the Halogen Moister Analyzer 20085. Thereafter the Biobeads® were subdivided into 2 bottles (bottle A and bottle B), water added to them and the bottles autoclaved, and subsequently stored at 2-8°C until used.

### Virosome formation by OEG removal with Biobeads®

Water was removed from batch chromatography bottle A using a syringe and needle. The supernatant with PL was transferred into bottle A, placed in a turbula shaker and shaken at 23 rpm for 1 hour at room temperature, to remove the OEG Water was removed from batch chromatography Bottle B as well. The suspension from bottle A was transferred to bottle B and the bottle placed in the turbula shaker and shaken at 23 rpm for 1 hour at RT to complete removal of the OEG. The virosomes formed (raw trivalent virosome) were collected, the HA content determined and thereafter diluted with PBS accordingly to yield HA concentration of ∼33 µg/mL. The trivalent virosome final bulk was filtrated using 0.22 µm syringe filter and stored at 2-8 °C until used.

Cryo-TEM pictures of the obtained virosomes showed optically-dense regions in the membrane indicating antigen incorporation into the virosomal membrane.

### Example 2 Stability testing

The virosomal trivalent final bulk was filled (50 mL/bag) in 6 Flexboy® bags (Sartorius AG) and 1 glass bottle (50 mL) and stored at 2-8°C up to 3 months. The samples were collected from the glass vial and 6 Flexboy bags after 3 months storage at 2-8°C and analyzed (Table 1) where it was observed that the stability was not changed during this period.

**Table 1: Stability study of trivalent virosome cocktail**

| **Assay** | **Product property** | **T = 0** | **T = 3 months (2-8°C)** |
|---|---|---|---|
| Appearance | Visual appearance | Slightly opalescent, | Slightly opalescent, |
| Particle Size | Particle size (nm) | 183 | 188 |
| Charge | Zeta potential (mV) | -8 | -8 |
| HPLC | HA content (ug/ml) | B. Bris. 53 | B. Bris. 59 |
| HA-SRID | HA content/in vitro potency (ug/ml) | 39 (A. Cal.); 89 (A. Vic.); 62 (B. Bris)* | 44 (A. Cal.); 83 (A. Vic.); 61 (B. Bris)* |
| | HA ratio | A. Cal., 0.2: A. Vic., 0.5: B. Bris, 0.3 | A. Cal., 0.2: A. Vic., 0.4: B. Bris, 0.3 |

| | | | |
|---|---|---|---|
| *The difference in ratio was due to the difference in starting ratio of the IVB's, which was theoretical: 0.2: 0.4: 0.3, respectively for A. Cal., A. Vic., and B. Bris. | | | |

## Claims

1. Method for preparing a virosome preparation comprising viral envelope protein of at least two, preferably at least three, more preferably at least four different enveloped viruses, comprising the steps of:
(a) providing at least two, preferably at least three, more preferably at least four enveloped viruses; and optionally inactivating said at least two, preferably at least three, more preferably at least four enveloped viruses;
(b) solubilizing the at least two, preferably at least three, more preferably at least four viral envelopes by adding a solubilizing agent; and
(c) reconstituting virosomal membranes comprising viral envelope protein of said at least two, preferably at least three, more preferably at least four different viruses by removing the solubilizing agent,
wherein the viral envelopes of said two, preferably at least three, more preferably at least four different virus are blended prior to reconstitution of the virosomal membranes.

2. Method according to claim 1, wherein the viral envelopes of said at least said two preferably at least three, more preferably at least four enveloped viruses are blended prior to inactivating the at least two, preferably at least three, more preferably at least four enveloped viruses.

3. Method according to claim 1, wherein the viral envelopes of said at least said two preferably at least three, more preferably at least four enveloped viruses are blended after inactivation of viruses and prior to solubilizing the viral envelopes.

4. Method according to claim 1, wherein the viral envelopes of said at least said two preferably at least three, more preferably at least four enveloped viruses are blended after solubilizing the viral envelopes.

5. Method according to any one of the preceding claims, wherein the at least two, preferably at least three, more preferably at least four different enveloped viruses comprise at least two, preferably at least three, more preferably at least four different virus strains of an enveloped virus.

6. Method according to claim 5, wherein the enveloped virus is an influenza virus and the viral envelope protein is hemagglutinin (HA) and/or neuraminidase (NA).

7. Method according to any one of the preceding claims, further comprising the step of adding one or more exogenous components.

## Patentansprüche

1. Verfahren zur Herstellung einer Virushüllprotein von wenigstens zwei, bevorzugt wenigstens drei, stärker bevorzugt wenigstens vier unterschiedlichen behüllten Viren umfassenden Virosomenpräparation, umfassend die Schritte:
(a) Bereitstellen von wenigstens zwei, bevorzugt wenigstens drei, stärker bevorzugt wenigstens vier behüllten Viren; und gegebenenfalls Inaktivieren der wenigstens zwei, bevorzugt wenigstens drei, stärker bevorzugt wenigstens vier behüllten Viren;
(b) Solubilisieren der wenigstens zwei, bevorzugt wenigstens drei, stärker bevorzugt wenigstens vier Virushüllen durch Zugeben eines Lösungsvermittlers; und
(c) Rekonstituieren von Virushüllprotein der wenigstens zwei, bevorzugt wenigstens drei, stärker bevorzugt wenigstens vier unterschiedlichen Viren umfassenden Virosomenmembranen durch Entfernen des Lösungsvermittlers,
wobei die Virushüllen der wenigstens zwei, bevorzugt wenigstens drei, stärker bevorzugt wenigstens vier unterschiedlichen Viren vor der Rekonstitution der Virosomenmembranen vermischt werden.

2. Verfahren nach Anspruch 1, wobei die Virushüllen der wenigstens zwei, bevorzugt wenigstens drei, stärker bevorzugt wenigstens vier behüllten Viren vor dem Inaktivieren der wenigstens zwei, bevorzugt wenigstens drei, stärker bevorzugt wenigstens vier behüllten Viren vermischt werden.

3. Verfahren nach Anspruch 1, wobei die Virushüllen der wenigstens zwei, bevorzugt wenigstens drei, stärker bevorzugt wenigstens vier behüllten Viren nach Inaktivierung von Viren und vor Solubilisieren der Virushüllen vermischt werden.

4. Verfahren nach Anspruch 1, wobei die Virushüllen der wenigstens zwei, bevorzugt wenigstens drei, stärker bevorzugt wenigstens vier behüllten Viren nach Solubilisieren der Virushüllen vermischt werden.

5. Verfahren nach einem der vorhergehenden Ansprüche, wobei die wenigstens zwei, bevorzugt wenigstens drei, stärker bevorzugt wenigstens vier unterschiedlichen behüllten Viren wenigstens zwei, bevorzugt wenigstens drei, stärker bevorzugt wenigstens vier unterschiedliche Virusstämme eines behüllten Virus umfassen.

6. Verfahren nach Anspruch 5, wobei es sich bei dem behüllten Virus um ein Influenzavirus und dem Virushüllprotein um Hämagglutinin (HA) und/oder Neuraminidase (NA) handelt.

7. Verfahren nach einem der vorhergehenden Ansprüche, ferner umfassend den Schritt der Zugabe einer oder mehrerer exogener Komponenten.

## Revendications

1. Procédé d'élaboration d'une préparation de virosome comprenant une protéine d'enveloppe virale d'au moins deux, préférentiellement d'au moins trois, plus préférentiellement d'au moins quatre virus enveloppés différents, comprenant les étapes consistant à :
(a) se munir d'au moins deux, préférentiellement d'au moins trois, plus préférentiellement d'au moins quatre virus enveloppés ; et éventuellement inactiver lesdits au moins deux, préférentiellement au moins trois, plus préférentiellement au moins quatre virus enveloppés ;
(b) solubiliser les au moins deux, préférentiellement au moins trois, plus préférentiellement au moins quatre enveloppes virales en ajoutant un agent de solubilisation ; et
(c) reconstituer les membranes virosomales comprenant une protéine d'enveloppe virale desdits au moins deux, préférentiellement au moins trois, plus préférentiellement au moins quatre virus différents en éliminant l'agent de solubilisation,
où les enveloppes virales desdits deux, préférentiellement au moins trois, plus préférentiellement au moins quatre virus différents sont mélangées avant reconstitution des membranes virosomales.

2. Procédé selon la revendication 1, où les enveloppes virales desdits au moins deux, préférentiellement au moins trois, plus préférentiellement au moins quatre virus enveloppés sont mélangées avant inactivation des au moins deux, préférentiellement au moins trois, plus préférentiellement au moins quatre virus enveloppés.

3. Procédé selon la revendication 1, où les enveloppes virales desdits au moins deux, préférentiellement au moins trois, plus préférentiellement au moins quatre virus enveloppés sont mélangées après inactivation du virus et avant solubilisation des enveloppes virales.

4. Procédé selon la revendication 1, où les enveloppes virales desdits au moins deux, préférentiellement au moins trois, plus préférentiellement au moins quatre virus enveloppés sont mélangées après solubilisation des enveloppes virales.

5. Procédé selon l'une quelconque des revendications précédentes, où les au moins deux, préférentiellement au moins trois, plus préférentiellement au moins quatre virus enveloppés différents comprennent au moins deux, préférentiellement au moins trois, plus préférentiellement au moins quatre souches virales différentes d'un virus enveloppé.

6. Procédé selon la revendication 5, où le virus enveloppé est un virus influenza et la protéine d'enveloppe virale est l'hémagglutinine (HA) et/ou la neuraminidase (NA).

7. Procédé selon l'une quelconque des revendications précédentes, comprenant en outre l'étape d'ajout d'un ou de plusieurs composants exogènes.
